# EUROPÄISCHE PATENTSCHRIFT

(11) **EP 1 843 706 B1**
(45) Veröffentlichungstag und Bekanntmachung des Hinweises auf die Patenterteilung: **01.07.2009**
(21) Anmeldenummer: 05784155.3
(22) Anmeldetag: 25.08.2005
(51) Int. Cl.: A61B 17/00, A61B 17/04

(54) **CHIRURGISCHE VORRICHTUNG ZUM HINDURCHFÜHREN WENIGSTENS ZWEIER NÄHFÄDEN DURCH DEN RANDBEREICH EINER GEWEBEÖFFNUNG**
SURGICAL DEVICE FOR GUIDING AT LEAST TWO SUTURE THREADS THROUGH THE MARGINAL AREA OF A TISSUE OPENING
DISPOSITIF CHIRURGICAL POUR GUIDER AU MOINS DEUX FILS DE SUTURE A TRAVERS LES BORDS D'UNE OUVERTURE DANS UN TISSU

(30) Priorität: 30.08.2004 DE 102004041936
(43) Veröffentlichungstag der Anmeldung: 17.10.2007
(73) Patentinhaber: Medi-Globe GmbH, 83101 Rohrdorf-Achenmühle (DE)
(72) Erfinder: SCHWARZ, Werner, 83324 Ruhpolding (DE)
(74) Vertreter: Nätebusch, Roderich
(86) Internationale Anmeldenummer: PCT/DE2005/001497
(87) Internationale Veröffentlichungsnummer: WO 2006/024272

(56) Entgegenhaltungen:
- DE-C1- 19 942 951
- US-A- 5 304 184
- US-A- 5 417 699
- US-A- 5 527 322

## Beschreibung

Die Erfindung bezieht sich auf eine chirurgische Vorrichtung zum Hindurchführen wenigstens zweier Nähfäden durch den Randbereich einer insbesondere in einer Arterie eines Individuums vorhandenen, gegebenenfalls durch Einschneiden gebildeten Gewebeöffnung und zum Zurückziehen der durch den betreffenden Randbereich hindurchgeführten jeweiligen Nähfädenenden aus der genannten Öffnung, mit einer stabförmigen Nadel- und Fadenführungseinrichtung, in der jeweils an Nadeln befestigte Nähfäden in Führungs- bzw. Aufnahmeöffnungen derart geführt sind, dass diese von einem am proximalen Vorrichtungsende angeordneten Nadelabgabebereich zu einem Nadelaufnahmebereich am distalen Vorrichtungsende hin über einen Zwischenraumbereich bewegbar sind, wobei der betreffende Zwischenraumbereich eine zumindest der Dicke der Wandung der Gewebeöffnung entsprechende Länge und eine bezogen auf die Außenform des Nadelabgabebereiches und/ oder des Nadelaufnahmebereiches für eine unbehinderte Bewegung der betreffenden Nadeln zwischen den betreffenden Nadelbereichen verminderte Außenform aufweist, und wobei die genannten Nadeln nach Hindurchführen durch den Randbereich der betreffenden Gewebeöffnung in den Nadelaufnahmebereich zur dortigen permanenten Aufnahme bewegbar sind und wobei danach die stabförmige Nadel- und Fadenführungseinrichtung zusammen mit den im Nadelaufnahmebereich permanent aufgenommenen Nadeln und den mit diesen verbundenen Nähfadenenden aus der genannten Gewebeöffnung wieder derart zurückziehbar sind, dass durch Zusammenziehen sämtlicher Nähfadenenden außerhalb der betreffenden Gewebeöffnung diese verschließbar ist.

Eine Vorrichtung der vorstehend genannten Art ist bereits bekannt (DE 199 42 951 C1 = EP 1 217 955), die als Basis für den Oberbegriff des Anspruchs 1 dienst. Bei dieser bekannten Vorrichtung ist die stabförmige Nadeln- und Fadenführungseinrichtung durch drei relativ zueinander verdrehbare Teile gebildet, und zwar durch ein am proximalen Vorrichtungsende befindliches Fadenzuführungsteil, ein am distalen Vorrichtungsende vorgesehenes Fadenaufnahmeteil und ein dazwischen liegendes mittleres Fadenfreigabe-/Fadenklemmteil. Das betreffende mittlere Fadenfreigabe-/Fadenklemmteil weist dabei einen solchen Querschnitt auf, dass es in zumindest einer Drehstellung die Einführung der von dem am proximalen Vorrichtungsende vorgesehenen Fadenzuführungsteil zugeführten Nähfäden in in dem am distalen Vorrichtungsende vorgesehenen Fadenaufnahmeteil freiliegende Aufnahmeöffnungen ermöglicht und in einer von dieser Drehstellung verschiedenen Drehposition die in den betreffenden Aufnahmeöffnungen zusammen mit den Nadeln aufgenommenen Nähfäden für ein Herausziehen der gesamten Fadenführungseinrichtung aus der genannten Öffnung heraus festzuhalten gestattet. Obwohl mit dieser bekannten Vorrichtung zufriedenstellend Öffnungen in einer Wandung einer Hülle, insbesondere in einer Arterienwand eines Individuums verschlossen werden können, besteht der Wunsch nach Bereitstellung einer einfacher zu bedienenden chirurgischen Vorrichtung zum Hindurchführung wenigstens zweier Nähfäden durch den Randbereich einer Gewebeöffnung, bei der ohne Drehbewegungen von Einzelteilen der Vorrichtung ausgekommen werden kann.

Es ist bereits eine Nähvorrichtung bekannt (WO 94/08516), bei der bis zu vier mit Nähfäden verbundene Nadeln mittels einer Schiebeeinrichtung durch eine Wandung eines Blutgefäßes nahe des Randbereiches einer darin befindlichen Öffnung hindurchgeführt und von einer innerhalb des betreffenden Blutgefäßes im Bereich der genannten Öffnung befindlichen Aufnahmeeinrichtung aufgenommen werden können. Diese Aufnahmeeinrichtung ist durch eine fangkorbartige Nadelaufnahmeeinrichtung gebildet, die zunächst im zusammengelegten Zustand durch die betreffende Öffnung hindurchgeführt und sodann in der betreffenden Öffnung aufgeweitet wird, um daraufhin am Randbereich der betreffenden Öffnung innerhalb des Blutgefäßes anzuliegen. Die durch den Randbereich der erwähnten Öffnung hindurchgeführten Nadeln sind von dieser fangkorbartigen Nadelaufnahmeeinrichtung aufnehmbar, die anschließend um ihre Längsachse verdreht wird, um die betreffenden Nadeln festzuhalten. Danach wird die betreffende fangkorbartige Nadelaufnahmeeinrichtung-mit den in ihr enthaltenen Nadeln zusammengelegt, um durch die erwähnte Öffnung vollständig herausgezogen zu werden. Bei diesem Vorgang werden die mit den Nadelenden verbundenen Nähfäden aus Vorratsmagazinen herausgezogen, durch die Einstichstellen der erwähnten Nadeln nahe des Randbereiches der erwähnten Öffnung hindurchgezogen und aus der betreffenden Öffnung wieder herausgezogen. Außerhalb der erwähnten Öffnung können die Nähfädenenden dann zusammengezogen und gegebenenfalls verknotet werden, um die betreffende Öffnung zu verschließen.

Obwohl die vorstehend betrachtete bekannte Vorrichtung vom Prinzip her einen relativ einfachen Aufbau zeigt, können sich jedoch bei der Aufnahme und beim Festhalten der nahe des Randbereiches der jeweiligen Öffnung hinduchgeführten Nadeln in der fangkorbartigen Nadelaufnahmeeinrichtung Probleme ergeben, wenn eine oder mehrere Nadeln durch diese Nadelaufnahmeeinrichtung nicht festgehalten werden können. In solchen Fällen sind dann komplizierte Eingriffe erforderlich, um die in dem Blutgefäß enthaltenen Nadeln wieder zu entfernen.

Es ist ferner eine Vorrichtung zum Hindurchführen zweier Nähfäden durch eine Wandung eines Blutgefäßes nahe des Randbereiches einer darin gebildeten Öffnung bekannt (WO 94/13211), die einen in die betreffende Öffnung einzuführenden Nadelträger und eine außerhalb der betreffenden Öffnung befindliche Nadelaufnahmeeinrichtung enthält. Der Nadelträger ist mit Nähfäden ausgestattet, deren Enden mit Nadeln verbunden sind. Im Gebrauch wird der erwähnte Nadelträger zunächst vollständig durch die erwähnte Öffnung in das zu verschließende Blutgefäß eingeführt, und anschließend werden die Nadeln durch den Randbereich der betreffenden Öffnung von innen nach außen hindurchgeführt. Dabei verbleiben die restlichen Nadelträgerteile zunächst noch in dem Blutgefäß. Anschließend werden diese Nadelträgerteile aus dem Blutgefäß durch die erwähnte Öffnung herausgezogen, so dass in dem betreffenden Blutgefäß lediglich der mit den bereits herausgeführten Nadeln verbundene Nähfaden verbleibt. Durch weiteres Zurückziehen der Nadeln wird der betreffende Nähfaden schließlich im Innern des betreffenden Blutgefäßes gespannt, so dass danach die Nähfädenenden verknotet werden können. Dadurch ist dann die betreffende Öffnung verschlossen.

Die gerade betrachtete bekannte Vorrichtung gestattet zwar vom Prinzip her Nähfäden durch die Wandung eines Blutgefäßes nahe des Randbereiches einer darin vorhandenen Öffnung hindurchzuführen; dabei ist jedoch auch hier die Sicherheit im Zusammenhang mit der Aufnahme der Nadelenden in der Nadelaufnahmeeinrichtung zumindest kritisch. Wird die eine oder andere Nadel nämlich nicht sicher von der Nadelaufnahmeeinrichtung aufgenommen, so sind auch in diesem Fall zusätzliche Eingriffe zur Vermeidung von Komplikationen notwendig. Aus Dimensionsgründen muss hier der Zugang zum Blutgefäß, und zwar insbesondere zur Arterie aufgeweitet werden. Damit ist aber die betreffende bekannte Vorrichtung nicht minimalinvasiv.

Es ist außerdem eine Vorrichtung zum Hindurchführen wenigstens eines Nähfadens durch die Wandung eines Blutgefäßes eines Individuums nahe des Randbereiches einer darin vorhandenen Öffnung bekannt (WO 95/13021, US 5.527.322, US 5.792.152). Diese bekannte Vorrichtung weist eine stabförmige Fadenführungseinrichtung auf, an deren Spitze ein Nasenteil mit einer Nadelumlenkbahn vorgesehen ist, welches über einen Bereich verminderten Querschnitts mit der stabförmigen Fadenführungseinrichtung verbunden ist. In dieser stabförmigen Fadenführungseinrichtung gibt es eine Nadelzuführöffnung, die zur Eintrittsseite der Nadelumlenkbahn im Nasenteil ausgerichtet ist. Außerdem gibt es in der stabförmigen Fadenführungseinrichtung eine zweite Nadelführungsöffnung, die zu der Austrittsseite der Nadelumlenkbahn im Nasenteil ausgerichtet ist. Aufgrund dieses Aufbaus funktioniert die gerade betrachtete bekannte Vorrichtung wie folgt. Zunächst wird die gesamte Vorrichtung in die zu verschließende Öffnung eines Blutgefäßes eines Individuums soweit eingeführt, dass die Wandung der betreffenden Öffnung an dem erwähnten Bereich verminderten Querschnitts anliegt, über den das Nasenteil mit der stabförmigen Fadenführungseinrichtung verbunden ist. Allerdings ist hier wegen des erwähnten Bereiches verminderten Querschnitts keine sichere und genaue Positionierung der betreffenden Vorrichtung am Randbereich des zu verschließenden Blutgefäßes möglich. Sodann wird eine mit einem Nähfaden verbundene Nadel durch die Nadelzuführöffnung der stabförmigen Fadenführungseinrichtung nach vorn zu dem Nasenteil hin bewegt, wobei die betreffende Nadel dabei die Wand des Blutgefäßes nahe des Randbereiches der erwähnten Öffnung durchsticht und anschließend in der Umlenkbahn des Nasenteiles derart umgelenkt wird, dass sie sodann den Randbereich der betreffenden Gefäßwand von innen nach außen durchsticht. Daraufhin wird die betreffende Nadel durch die erwähnte weitere Nadelführungsöffnung der stabförmigen Fadenführungseinrichtung wieder zurückgeführt, so dass damit der Randbereich der erwähnten Öffnung an zwei diametral gegenüberliegenden Stellen nunmehr von einem Faden durchzogen ist. Dieser Faden muss dann anschließend aus der Umlenkbahn über eine mit dieser verbundene Fadenfreigabe-Schlitzanordnung herausgeführt werden, so dass daraufhin die gesamte Vorrichtung aus der Öffnung des erwähnten Blutgefäßes herausgezogen werden kann.

Die vorstehend betrachtete bekannte Vorrichtung gestattet zwar vom Prinzip her wenigstens einen Nähfaden durch die Wand eines Blutgefäßes nahe des Randbereiches einer darin vorhandenen Öffnung an zwei diametral gegenüberliegenden Stellen hindurchzuführen; die erwähnte Nadelumlenkkonstruktion bereitet jedoch in der Praxis zuweilen Probleme, da infolge der relativ starken Krümmung der in dem erwähnten Nasenteil vorhandenen Umlenkbahn nur flexible Nadeln oder kleine Nadeln verwendbar sind, was indessen Probleme beim Hindurchführen solcher Nadeln durch Gefäßwände verursachen kann.

Es ist schließlich auch schon eine Nähvorrichtung zum Hindurchführen der Enden eines Nähfadens durch die Wand eines Blutgefäßes eines Individuums nahe des Randbereiches einer darin vorhandenen Öffnung bekannt (US 5.860.990). Bei dieser bekannten Vorrichtung wird ein Nähfadenvorrat mit schlaufenförmigen Fadenenden mittels einer stabförmigen Fadenzuführeinrichtung durch die betreffende Öffnung in das Blutgefäß eingeführt. Außerhalb der betreffenden stabförmigen Fadenzuführeinrichtung sind an zwei diametral gegenüberliegenden Stellen nadelförmige Fadenaufnehmer vorgesehen, die nach Durchstechen der Gefäßwand nahe des Randbereiches der betreffenden Öffnung die schlaufenförmig ausgebildeten Fadenenden aufnehmen und dann nach außen aus dem Blutgefäß herausziehen sollen.

Obwohl die zuletzt betrachtete bekannte Vorrichtung vom Prinzip her schlaufenförmig ausgebildete Enden eines Nähfadens durch eine Blutgefäßwand nahe des Randbereiches einer darin vorhandenen Öffnung hindurchzuführen gestattet, zeigt indessen auch diese Konstruktion bei ihrem Einsatz Probleme mit der Aufnahme der erwähnten schlaufenförmigen Fadenenden. Eine sichere Aufnahme der betreffenden schlaufenförmigen Fadenenden ist nämlich nur dann gewährleistet, wenn die betreffenden Fadenenden von der stabförmigen Fadenzuführeinrichtung in einer solchen definierten Position festgehalten werden, in der die nadelförmigen Fadenaufnehmer diese schlaufenförmigen Fadenenden auch erfassen können. Dies ist in der Praxis zuweilen nur mit erheblichen Schwierigkeiten erreichbar.

Der Erfindung liegt die Aufgabe zugrunde, eine chirurgische Vorrichtung der eingangs genannten Art so weiterzubilden, dass ohne drehbare Elemente beim Hindurchführen wenigstens zweier Nähfäden durch den Randbereich einer Gewebeöffnung ausgekommen werden kann und zugleich eine sichere Positionierung der betreffenden Vorrichtung in der betreffenden Gewebeöffnung ermöglicht ist.

Gelöst wird die vorstehend aufgezeigte Aufgabe bei einer Vorrichtung der eingangs genannten Art erfindungsgemäß dadurch, dass der Nadelabgabebereich, der Zwischenraumbereich und der Nadelaufnahmebereich der stabförmigen Nadel- und Fadenführungseinrichtung starr miteinander verbunden sind,
dass die stabförmige Nadel- und Fadenführungseinrichtung von einem deren Zwischenraumbereich formschlüssig umgebenden länglichen Außenglied verschließbar ist, welches unter Freigabe des genannten Zwischenraumbereiches relativ zu der stabförmigen Fadenführungseinrichtung in deren Längsrichtung verschiebbar ist,
und dass der Nadelabgabebereich zum distalen Vorrichtungsende hin und das längliche Außenglied in seinem Bereich zum proximalen Vorrichtungsende hin einander zugewandte Endseiten aufweisen.

Die Erfindung zeichnet sich gegenüber den bisher bekannten Vorrichtungen zum Verschließen einer Gewebeöffnung eines Individuums durch den Vorteil aus, dass mit insgesamt besonders geringem Aufwand eine sichere und genaue Positionierung der betreffenden Vorrichtung in der durch wenigstens zwei Nähfäden zu verschließenden Gewebeöffnung ermöglicht ist, ohne dass dabei drehbare Elemente erforderlich sind. Gemäß der vorliegenden Erfindung ist nämlich lediglich eine starre, also keine drehbaren Elemente aufweisende stabförmige Nadel- und Fadenführungseinrichtung vorgesehen, mit der sich die erwünschte sichere und genaue Positionierung der Vorrichtung in der zu verschließenden Gewebeöffnung unter Ausnutzung des distalen Endes des Nadelabgabebereiches und des dem proximalen Vorrichtungsende zugewandten Randbereiches des vorhandenen länglichen Außengliedes erreichen lässt, wenn dieses Außenglied unter Freigabe des Zwischenraumbereiches zwischen dem Nadelabgabebereich und dem Nadelaufnahmebereich der erfindungsgemäßen Vorrichtung in Richtung zum distalen Vorrichtungsende hin bewegt wird. In diesem Falle klemmt sich nämlich der Randbereich der durch Nähfäden zu verschließenden Gewebeöffnung in den erwähnten Zwischenraumbereich der erfindungsgemäßen Vorrichtung zwischen deren Fadenabgabebereich und Fadenaufnahmebereich ein, womit die Stelle genau und sicher festgelegt ist, in der anschließend die Nadeln mit den daran befestigten Nähfadenenden durch den erwähnten Randbereich der Gewebeöffnung geführt werden können.

Zweckmäßigerweise verlaufen die Endseiten des Nadelabgabebereiches am distalen Vorrichtungsende und des länglichen Außengliedes in seinem Bereich zum proximalen Vorrichtungsende hin parallel zueinander, und vorzugsweise verlaufen die betreffenden Endseiten rechtwinklig zur Längsrichtung der stabförmigen Fadenführungseinrichtung. Dadurch ergibt sich eine besonders einfache und sichere Anlage der betreffenden Endseiten auf den beiden Seiten des Randbereiches der zu verschließenden Gewebeöffnung.

Von Vorteil ist es ferner, wenn gemäß weiterer Ausbildung der Erfindung die Nadelaufnahmeöffnungen des Nadelaufnahmebereichs in ihrem jeweiligen Nadeleinlassbereich trichterförmig ausgebildet sind. Dies erleichtert in vorteilhafter Weise die Einführung der Nadeln in die betreffenden Nadelaufnahmeöffnungen.

Eine besonders vorteilhafte Ausgestaltung der Vorrichtung gemäß der Erfindung ergibt sich dadurch, dass der Nadelaufnahmebereich auf seiner dem proximalen Vorrichtungsende zugewandten Seite eine von seinem Außenumfang in Richtung zur Vorrichtungsmittelachse verlaufenden Abschrägung aufweist, die vorzugsweise einen Winkel von ≤45° bezogen auf die Vorrichtungsmittelachse bildet. Durch diese Maßnahme ist ein leichtes Herausziehen der Vorrichtung gemäß der Erfindung aus der jeweiligen zu verschließenden Gewebeöffnung möglich, nachdem das längliche Außenglied, welches zunächst den Zwischenraumbereich zwischen dem Nadelabgabebereich und dem Nadelaufnahmebereich der Nadel- und Fadenführungseinrichtung verschlossen hat, zum distalen Vorrichtungsende hin in eine den betreffenden Zwischenraum völlig freigebende Stellung bewegt ist.

Zweckmäßigerweise weist die stabförmige Nadel- und Fadenführungseinrichtung in ihrem Zwischenraumbereich eine Durchgangsöffnung auf, die mit einer in der stabförmigen Nadel- und Fadenführungseinrichtung vorhandenen und zu deren proximalen Ende hin verlaufenden Anschlussöffnung verbunden ist. Durch diese Maßnahme lässt sich in vorteilhafter Weise besonders einfach zusätzlich feststellen, wann die Vorrichtung gemäß der Erfindung in der jeweils zu verschließenden Gewebeöffnung richtig positioniert ist. Bei richtiger Positionierung der betreffenden Vorrichtung kann nämlich in der Gewebeöffnung enthaltene Gewebeflüssigkeit, wie Blut, durch die erwähnte Durchgangsöffnung und die mit dieser verbundene Anschlussöffnung zum proximalen Vorrichtungsende hin gelangen und damit eine zusätzliche optische Anzeige für die richtige Positionierung der gesamten Vorrichtung liefern.

Zweckmäßigerweise weist das längliche Außenglied ebenfalls eine Durchgangsöffnung auf, die in der den genannten Zwischenraumbereich der stabförmigen Nadel- und Fadenführungseinrichtung verschließenden Stellung des länglichen Außengliedes mit der in dem Zwischenraumbereich der stabförmigen Nadel- und Fadenführungseinrichtung befindlichen Durchgangsöffnung verbunden ist. Dadurch lässt sich auf besonders einfache Weise bei durch das längliche Außenglied völlig geschlossenem Zwischenraumbereich der stabförmigen Nadel- und Fadenführungseinrichtung bei deren Einführen in die zu verschließende jeweilige Gewebeöffnung diejenige Stelle feststellen, an der der betreffende Zwischenraumbereich den Randbereich der betreffenden Gewebeöffnung aufzunehmen imstande ist, wenn anschließend das genannte längliche Außenglied zur Freigabe des Zwischenraumbereichs zum distalen Vorrichtungsende hin verschoben wird.

Zweckmäßigerweise weist die stabförmige Nadel- und Fadenführungseinrichtung einen ovalen Querschnitt auf, und der Nadelabgabebereich und der Nadelaufnahmebereich sind dabei lediglich nahe der Endbereiche auf der größeren Querschnittsachse des betreffenden ovalförmigen Querschnitts angeordnet. Hierdurch ist in vorteilhafter Weise eine relativ geringe Beanspruchung der jeweils zu verschließenden Gewebeöffnung durch deren Querschnittserweiterung nach dem Einführen der stabförmigen Nadel- und Fadenführungseinrichtung sichergestellt.

Gemäß einer anderen zweckmäßigen Weiterbildung der vorliegenden Erfindung kann die stabförmige Nadel- und Fadenführungseinrichtung jedoch auch einen kreisförmigen Querschnitt oder einen an diesen angenäherten Querschnitt aufweisen, und der Nadelabgabebereich und der Nadelaufnahmebereich können mit entsprechenden Nadelführungsöffnungen bzw. Nadelaufnahmeöffnungen um den betreffenden Querschnitt herum verteilt angeordnet sein. Dies ermöglicht auf besonders einfache weise, eine Vielzahl von Nadeln mit zugehörigen Nähfadenenden von dem Nadelabgabebereich zum Nadelaufnahmebereich beispielsweise gleichzeitig abzugeben.

Um das erwähnte längliche Außenglied auf der stabförmigen Nadel- und Fadenführungseinrichtung in deren Längsrichtung bequem verschieben zu können, ist das betreffende längliche Außenglied mit einer innerhalb oder außerhalb der stabförmigen Nadel- und Fadenführungseinrichtung vorgesehenen Schiebereinrichtung verbunden und mittels dieser relativ zu der stabförmigen Nadel- und Fadenführungseinrichtung in deren Längsrichtung verschiebbar.

Anhand von Zeichnungen wird die Erfindung nachstehend an einem Ausführungsbeispiel näher erläutert.
- Fig. 1: zeigt in einer schematischen Halbschnittdarstellung und in stark vergrößertem Maßstab eine chirurgische Vorrichtung gemäß einer Ausführungsform der vorliegenden Erfindung.
- Fig. 2, 3 und 4: zeigen in perspektivischen Ausschnitten und ebenfalls in stark vergrößerten Maßstäben jeweils einen Teil der in Fig. 1 dargestellten chirurgischen Vorrichtung in verschiedenen Arbeitsstellungen.

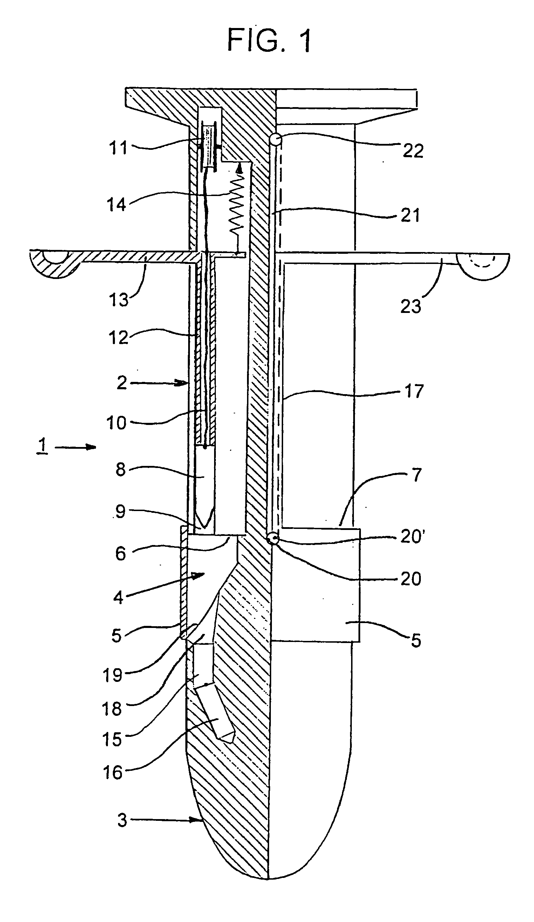

Die in Fig. 1 in einer schematischen Halbschnittdarstellung und in stark vergrößertem Maßstab dargestellte chirurgische Vorrichtung gemäß einer Ausführungsform der vorliegenden Erfindung besteht aus einer stabförmigen Nadel- und Fadenführungseinrichtung 1, welche einen am proximalen Vorrichtungsende, das der in Fig. 1 oben dargestellte Teil, vorgesehenen Nadelabgabebereich 2, einen am distalen Vorrichtungsende, das ist der in Fig. 1 unten dargestellte Teil, vorgesehenen Nadelaufnahmebereich 3 und einen zwischen dem Nadelabgabebereich 2 und dem Nadelaufnahmebereich 3 vorgesehenen Zwischenraumbereich 4 enthält. Diese drei Bereiche 2, 3 und 4 der dargestellten Vorrichtung sind starr miteinander verbunden. In Fig. 1 sind diese drei Bereiche 2, 3 und 4 zwar als aus einem Materialstück bestehend dargestellt, beispielsweise aus einem Kunststoff, wie zum Beispiel PEEK (Polyetheretherketon), oder aus Metall, wie aus Titan oder einer Titanlegierung; es versteht sich jedoch, dass die betreffenden Bereiche auch aus einzelnen Teilen hergestellt sein können, die dann fest bzw. starr miteinander verbunden sind, wie beispielsweise durch Schraubverbindungen.

Im Hinblick auf den erwähnten Maßstab der dargestellten Vorrichtung sei hier angemerkt, dass die Außendurchmesser des Nadelabgabebereiches 2 und des Nadelaufnahmebereiches 3 in der Praxis jeweils 3 mm bis 4 mm betragen können.

Der Zwischenraumbereich 4 der stabförmigen Nadel- und Fadenführungseinrichtung 1, auf den weiter unten noch näher eingegangen wird, ist gemäß Fig. 1 von einem länglichen Außenglied 5 formschlüssig umgeben, bei dem es sich beispielsweise um ein dünnes Blechteil, z.B. aus einem Kunststoff oder Metall, handeln kann, welches am Außenumfang der stabförmigen Nadel- und Fadenführungseinrichtung 1 fest anliegt, jedoch in Längsrichtung der betreffenden Nadel- und Fadenführungseinrichtung 1 verschiebbar ist, worauf weiter unten noch näher eingegangen wird. Durch das erwähnte längliche Außenglied 5 ist, wie aus Fig. 1 ersichtlich ist, der Zwischenraumbereich 4 zur Außenseite der stabförmigen Nadel- und Fadenführungseinrichtung 1 völlig verschließbar.

Der Nadelabgabebereich 2 weist als hier rotationssymmetrisch ausgebildeter Bereich zum distalen Vorrichtungsende hin eine Endseite 6 auf, und das längliche Außenglied 5 weist in seinem Bereich zum proximalen Vorrichtungsende hin ebenfalls eine Endseite 7 auf; diese beiden Endseiten 6 und 7 sind gewissermaßen einander zugewandt und verlaufen parallel zueinander; im vorliegenden Fall verlaufen sie im übrigen rechtwinklig zur Längsrichtung der Nadel- und Fadenführungseinrichtung 1.

Der Nadelabgabebereich 2 ist in seinem geschnitten dargestellten Teil und auch in seinem nicht geschnitten dargestellten Teil jeweils mit einer Nadel 8 versehen, die in einer Nadelführungsöffnung 9 aufgenommen ist und mit der das eine Ende eines Nähfadens 10 verbunden ist, der von einer am proximalen Vorrichtungsende vorgesehenen Vorratsrolle 11 geliefert wird.

In Fig. 1 ist noch angedeutet, dass auf dem proximalen Ende der sichtbaren Nadel 8 ein Nadelschieber 12 sitzt, der durch einen Betätigungshebel 13 betätigbar ist, welcher sich nahe des proximalen Vorrichtungsendes befindet. Eine Zugfeder 14 hält den Betätigungshebel 13 und damit den Nadelschieber 12 in der in Fig. 1 angedeuteten Stellung, in der sich die Nadel 8 in dem Nadelabgabebereich 2 befindet. Der betreffende Betätigungshebel 13 kann selbstverständlich auch für einen entsprechenden Nadelschieber und eine entsprechende Nadel in den nicht geschnitten dargestellten Bereich des Nadelabgabebereiches 2 wirksam sein.

Der am distalen Vorrichtungsende vorgesehene Nadelaufnahmebereich 3 enthält für die aus dem Nadelabgabebereich 2 abgebbaren Nadeln, wie für die dargestellte Nadel 8, Aufnahmeöffnungen, wie die in Fig. 1 dargestellte Aufnahmeöffnung 15, die mit der im Nadelabgabebereich 2 vorgesehenen zugehörigen Nadelführungsöffnung 9 fluchtet. In Fig. 1 ist die Nadelaufnahmeöffnung 15 noch mit einer abgeknickten Abschlussöffnung 16 verbunden, die unter einem leichten Winkel in Bezug auf die Aufnahmeöffnung 15 verläuft. Durch diese Maßnahme wird hier sichergestellt, dass die in den Nadelaufnahmebereich 3 eingeführte Nadel 8 sicher in diesem festgeklemmt wird und somit permanent im Nadelaufnahmebereich 3 festgehalten wird. Zum proximalen Ende hin ist die Aufnahmeöffnung 15 mit einem trichterförmigen Nadeleinlassbereich 18 ausgestattet, der ein leichteres Einführen der Nadel 8 in die Aufnahmeöffnung 15 ermöglicht. Dieses Merkmal ist von besonderem Vorteil bei Verwendung von Nadeln, die kürzer sind als die in Fig. 1 dargestellte Nadel 8.

Der zwischen dem Nadelabgabebereich 2 und dem Nadelaufnahmebereich 3 vorgesehene Zwischenraumbereich 4 weist eine zumindest der Dicke der Wandung einer Gewebeöffnung, die mittels der betreffenden Vorrichtung zu verschließen ist, entsprechende Länge und eine bezogen auf die Außenform des Nadelabgabebereiches 2 und/oder des Nadelaufnahmebereiches 3 für eine unbehinderte Bewegung der jeweiligen Nadeln, wie der Nadel 8, zwischen den betreffenden Nadelbereichen 2, 3 verminderte Außenform auf. In Fig. 1 ist dargestellt, dass der betreffende Zwischenraumbereich 4 in Bezug auf den Nadelabgabebereich 2 und den Nadelaufnahmebereich 3 gewissermaßen eingeschnürt ist. Darüber hinaus weist der Nadelaufnahmebereich 3 auf seiner dem proximalen Vorrichtungsende zugewandten Seite eine von seinem Außenumfang in Richtung zur Vorrichtungsmittelachse verlaufende Abschrägung 19 auf. Diese Abschrägung 19 verläuft also vom distalen Vorrichtungsende zum proximalen Vorrichtungsende hin. Die betreffende Abschrägung 19 weist vorzugsweise einen Winkel von ≤45° bezogen auf die Vorrichtungsmittelachse auf.

Bei der in Fig. 1 dargestellten Vorrichtung gemäß einer Ausführungsform der Erfindung ist der Zwischenraumbereich 4 nahe des Randes zum Nadelabgabebereich 2 mit einer Durchgangsöffnung 20 versehen, die hier zugleich mit einer deckungsgleichen Durchgangsöffnung 20' des länglichen Außengliedes 5 zusammenfällt. Die Durchgangsöffnung 20 ist im vorliegenden Fall mit einer im Innern des Nadelabgabebereichs 2 vorgesehenen Anschlussöffnung 21 mit einer am proximalen Vorrichtungsende vorgesehenen Austrittsöffnung 22 versehen, die gegebenenfalls mit einer Anzeigeeinrichtung, wie einem Schauglas, versehen sein kann. Auf die Bedeutung der Durchgangsöffnung 20 und der damit verbundenen Anschlussöffnung 21 sowie der Austrittsöffnung 22 wird weiter unten noch näher eingegangen werden.

Im Hinblick auf das längliche Außenglied 5 sei hier noch angemerkt, dass gemäß Fig. 1 dieses Außenglied mit einem Stegteil 17 mit einem am proximalen Vorrichtungsende angedeuteten Betätigungshebel 23 verbunden ist, mit dessen Hilfe das längliche Außenglied 5 in Längsrichtung der Nadel- und Fadenführungseinrichtung 1 relativ zu dieser verschiebbar ist. Im vorliegenden Fall erfolgt eine Verschiebung aus der in Fig. 1 dargestellten Stellung lediglich zum distalen Vorrichtungsende hin. Im übrigen könnte die Verschiebung des länglichen Außengliedes 5 auch durch ein im Innern der stabförmigen Nadel- und Fadenführungseinrichtung 1 geführten, zum proximalen Vorrichtungsende verlaufenden und mit dem betreffenden Außenglied 5 verbundenen Schiebeeinrichtung erfolgen.

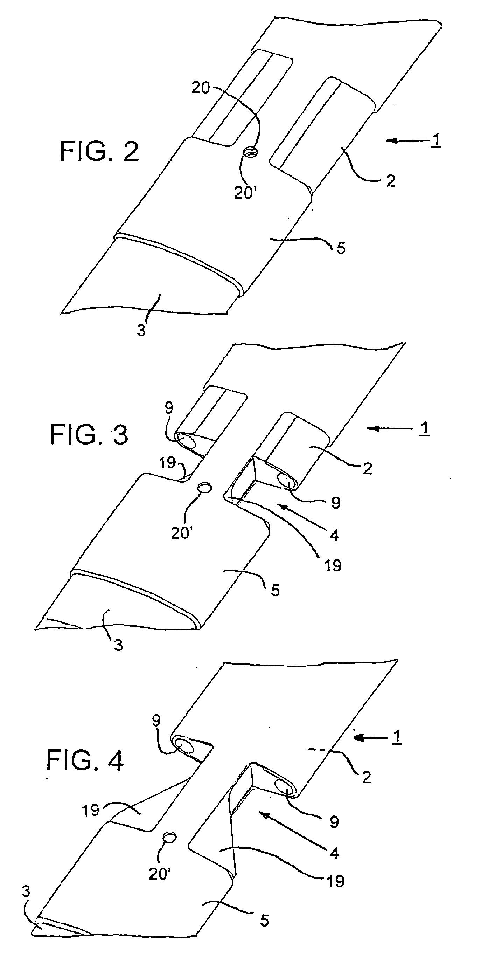

Nachdem zuvor der grundsätzliche Aufbau einer Ausführungsform der chirurgischen Vorrichtung gemäß der Erfindung anhand des in Fig. 1 gezeigten Teilschnittbildes erläutert worden ist, sei nunmehr unter Bezugnahme auf die Fig. 2, 3 und 4 auf die besonderen Effekte eingegangen, die mit dem Einsatz dieser Vorrichtung in der Praxis verbunden sind.

Zunächst sei generell unter Bezugnahme auf die Fig. 2 bis 4 angemerkt, dass dort eine gegenüber der in Fig. 1 dargestellten Vorrichtung hinsichtlich des länglichen Außengliedes 5 leicht modifizierte stabförmige Nadel- und Fadenführungseinrichtung 1 als einen ovalen Querschnitt aufweisend dargestellt ist, wobei der Nadelabgabebereich 2 und der Nadelaufnahmebereich 3 lediglich nahe der Endbereiche auf der größeren Querschnittsachse des betreffenden ovalen Querschnittsbereiches angeordnet sind. Dies bedeutet, dass Nadelabgabeöffnungen 9 gemäß Fig. 1 und zugehörige Nadelaufnahmeöffnungen 15 gemäß Fig. 1 auf der größeren Querschnittsachse des betreffenden ovalförmigen Querschnitts liegen. Dies ist in den Fig. 3 und 4 bezüglich der Nadelabgabeöffnungen 9 klar dargestellt.

Die in Fig. 2 ausschnittweise dargestellte Perspektivansicht zeigt die chirurgische Vorrichtung gemäß einer Ausführungsform der vorliegenden Erfindung entsprechend der in Fig. 1 dargestellten Stellung, also in einer Stellung, in der der Zwischenraumbereich 4 durch das längliche Außenglied 5 völlig verschlossen ist. Aus Fig. 2 ist überdies ersichtlich, dass die im Zusammenhang mit Fig. 1 erwähnte Durchgangsöffnung 20 des Zwischenraumbereiches 4 sich mit der Durchgangsöffnung 20' des länglichen Außengliedes in Deckung befindet. In der in Fig. 2 gezeigten Stellung wird die betreffende chirurgische Vorrichtung in eine zu verschließende Gewebeöffnung, wie die einer Arterie eingeführt, und zwar soweit, bis die Durchgangsöffnungen 20, 20' sich innerhalb der betreffenden Gewebeöffnung befinden und damit eine entsprechende Gewebeflüssigkeit durch diese Durchgangsöffnungen und die im Zusammenhang mit Fig. 1 erwähnte Anschlussöffnung 21 zur Austrittsöffnung 22 gelangen kann.

Fig. 3 zeigt die in Fig. 2 ausschnittweise in einer Perspektivansicht dargestellte chirurgische Vorrichtung in einer Stellung, in der das längliche Außenglied 5 zum distalen Vorrichtungsende hin verschoben ist. Nunmehr sind die Durchgangsöffnungen, von denen in Fig. 3 jetzt lediglich die Durchgangsöffnung 20' sichtbar ist, nicht mehr in Deckung miteinander, sondern der Zwischenraumbereich 4 ist leicht geöffnet. In diesem Falle kann der Randbereich der zu verschließenden Gewebeöffnung von diesem Zwischenraumbereich 4 aufgenommen werden, wobei die einander zugewandten Endseiten, nämlich die Endseite 6 des Nadelabgabebereiches 2 und die Endseite 7 des länglichen Außengliedes 5 gewissermaßen eine Fixierung der gesamten chirurgischen Vorrichtung und damit deren genaue und sichere Positionierung an dem Randbereich der zu verschließenden Gewebeöffnung vornehmen können. Ein Hin- und Herbewegen der gesamten chirurgischen Vorrichtung mit dem in seinem Zwischenraumbereich 4 aufgenommenen Randbereich der betreffenden Gewebeöffnung führt im übrigen nicht dazu, dass die gesamte Vorrichtung aus der betreffenden Gewebeöffnung herausgleitet. Vielmehr ist durch die erwähnten einander zugewandten Endseiten 6 und 7 eine genaue und sichere Positionierung und Fixierung der gesamten Vorrichtung am Randbereich der zu verschließenden Gewebeöffnung sichergestellt.

In der in Fig. 3 dargestellten Stellung der chirurgischen Vorrichtung werden zweckmäßigerweise die in den Nadelabgabeöffnungen 9 enthaltenen Nadeln, wie die in Fig. 1 dargestellte Nadel 8, mit den mit ihnen verbundenen Nähfäden in den Nadelaufnahmebereich 3 eingebracht. Das längliche Außenglied 5, das in dieser Stellung noch einen Teil des Zwischenraumbereiches 4 umgibt, trägt dabei dazu bei, dass die Nadeln sicher in ihre entsprechenden Nadelaufnahmeöffnungen, wie die Aufnahmeöffnung 15 gemäß Fig. 1, eingeführt werden können. Unterstützt wird diese Nadeleinführung im übrigen durch die im Zusammenhang mit Fig. 1 erwähnte trichterförmige Ausbildung der Nadeleinführung im Nadelaufnahmebereich 3.

In der in Fig. 4 dargestellten Stellung der chirurgischen Vorrichtung gemäß der Erfindung ist das längliche Außenglied 5 soweit zum distalen Vorrichtungsende hin verschoben, dass der Zwischenraumbereich 4 vollständig freigelegt ist. In dieser Stellung kann die betreffende chirurgische Vorrichtung ungehindert aus der Gewebeöffnung herausgezogen werden, durch deren Randbereiche zuvor Nadeln, wie die in Fig. 1 dargestellte Nadel 8 mit entsprechenden Nähfäden hindurchgeführt und im Nadelaufnahmebereich 3 permanent aufgenommen sind. Die betreffenden Nadeln sind in dem Nadelaufnahmebereich 3 so festgeklemmt, dass auch beim Zurückziehen der betreffenden Vorrichtung aus der Gewebeöffnung die Nadeln weiterhin in dem Nadelaufnahmebereich 3 verbleiben, jedoch die mit diesen verbundenen Nähfäden aus der genannten Gewebeöffnung wieder zurückziehbar sind, so dass diese Nähfädenenden und die jeweils anderen Nähfädenenden der betreffenden Nähfäden außerhalb der betreffenden Gewebeöffnung zum Verschließen dieser Gewebeöffnung verknotet werden können.

Im Hinblick auf die in den Fig. 2, 3 und 4 dargestellten Verhältnisse ergibt sich somit zusammenfassend, dass Fig. 2 die chirurgische Vorrichtung in einer Einführstellung bei der Einführung in eine zu verschließende Gewebeöffnung zeigt, durch deren Randbereich zumindest zwei Nähfäden hindurchzuführen sind; Fig. 3 zeigt die betreffende chirurgische Vorrichtung in einer Stellung, in der deren Nadeln mit den mit diesen verbundenen Nähfäden durch den Randbereich der zu verschließenden Gewebeöffnung geführt werden; Fig. 4 zeigt schließlich die betreffende Vorrichtung in der Stellung, in der diese aus der betreffenden Gewebeöffnung komplett herausziehbar ist bzw. herausgezogen wird. Dabei ist der Randbereich der betreffenden Gewebeöffnung hier von zwei Nähfäden durchzogen, deren Enden unter Verschließen der betreffenden Gewebeöffnung außerhalb dieser zusammengezogen und verknotet werden können.

Abschließend sei noch angemerkt, dass in den Fig. 2 bis 4 die stabförmige Nadel- und Fadenführungseinrichtung 1 als einen ovalförmigen Querschnitt aufweisend dargestellt ist. Gemäß der Erfindung ist es aber auch möglich, dass die betreffende Nadel- und Fadenführungseinrichtung 1 einen kreisförmigen Querschnitt oder einen an diesen angenäherten Querschnitt aufweist und dass der Nadelabgabebereich 2 und der Nadelaufnahmebereich 3 um den betreffenden Querschnitt herum mit einer entsprechenden Vielzahl von Nadelabgabeöffnungen bzw. Nadelaufnahmeöffnungen (mit n ≥ 2) verteilt angeordnet sind.

### Bezugszeichenliste

- 1: Nadel- und Fadenführungseinrichtung
- 2: Nadelabgabebereich
- 3: Nadelaufnahmebereich
- 4: Zwischenraumbereich
- 5: längliches Außenglied
- 6: Endseite
- 7: Endseite
- 8: Nadel
- 9: Nadelführungsöffnung
- 10: Nähfaden
- 11: Nähfaden
- 12: Nadelschieber
- 13: Betätigungshebel
- 14: Zugfeder
- 15: Aufnahmeöffnung
- 16: Abschlussöffnung
- 17: Stegteil
- 18: Nadeleinlassbereich
- 19: Abschrägung
- 20: Durchgangsöffnung
- 20': Durchgangsöffnung
- 21: Anschlussöffnung
- 22: Austrittsöffnung
- 23: Betätigungshebel

## Patentansprüche

1. Chirurgische Vorrichtung zum Hindurchführen wenigstens zweier Nähfäden (10) durch den Randbereich einer insbesondere in einer Arterie eines Individuums vorhandenen, gegebenenfalls durch Einschneiden gebildeten Gewebeöffnung und zum Zurückziehen der durch den betreffenden Randbereich hindurchgeführten jeweiligen Nähfädenenden-aus der genannten Öffnung, mit einer stabförmigen Nadel- und Fadenführuncrseinrichtug (1), in der jeweils an Nadeln (8) befestigte Nähfäden (10) in Führungs- bzw. Aufnahmeöffnungen (15) derart geführt sind, dass diese von einem am proximalen Vorrichtungsende angeordneten Nadelabgabebereich (2) zu einem Nadelaufnahmebereich (3) am distalen Vorrichtungsende hin über einen Zwischenraumbereich (4) bewegbar sind, wobei der betreffende Zwischenraumbereich (4) eine zumindest der Dicke der Wandung der Gewebeöffnung entsprechende Länge und eine bezogen auf die Außenform des Nadelabgabebereiches (2) und/oder des Nadelaufnahmebereiches (3) für eine unbehinderte Bewegung der betreffenden Nadeln (8) zwischen den betreffenden Nadelbereichen verminderte Außenform aufweist,
wobei die genannten Nadeln (8) nach Hindurchführen durch den Randbereich der betreffenden Gewebeöffnung in den Nadelaufnahmebereich (3) zur dortigen permanenten Aufnahme bewegbar sind und wobei danach die stabförmige Nadel- und Fadenführungseinrichtung (1) zusammen mit den im Nadelaufnahmebereich (3) permanent aufgenommenen Nadeln (8) und den mit diesen verbundenen Nähfadenenden aus der genannten Gewebeöffnung wieder derart zurückziehbar sind, dass durch Zusammenziehen sämtlicher Nähfadenenden außerhalb der betreffenden Gewebeöffnung diese verschließbar ist,
**dadurch gekennzeichnet, dass** der Nadelabgabebereich (2), der Zwischenraumbereich (4) und der Nadelaufnahmebereich (3) der stabförmigen Nadel- und Fadenführungseinrichtung (1) starr miteinander verbunden sind, dass die stabförmige Nadel- und Fadenführungseinrichtung (1) von einem deren Zwischenraumbereich (4) formschlüssig umgebenden länglichen Außenglied (5) verschließbar ist, welches unter Freigabe des genannten Zwischenraumbereiches (4) relativ zu der stabförmigen Nadel- und Fadenführungseinrichtung (1) in deren Längsrichtung verschiebbar ist,
und dass der Nadelabgabebereich (2) zum distalen Vorrichtungsende hin und das längliche Außenglied (5) in seinem Bereich zum proximalen Vorrichtungsende hin jeweils einander zugewandte Endseiten (6, 7) aufweisen.

2. Vorrichtung nach Anspruch 1, **dadurch gekennzeichnet, dass** die betreffenden Endseiten (6, 7) parallel zueinander oder rechtwinklig zur Längsrichtung der stabförmigen Nadel- und Fadenführungseinrichtung (1) verlaufen.

3. Vorrichtung nach Anspruche 1 oder 2,
**dadurch gekennzeichnet, dass** die Nadelaufnahmeöffnungen (15) des Nadelaufnahmebereiches (3) in ihrem jeweiligen Nadeleinlassbereich (18) trichterförmig ausgebildet sind.

4. Vorrichtung nach einem der Ansprüche 1 bis 3,
**dadurch gekennzeichnet, dass** der Nadelaufnahmebereich (3) auf seiner dem proximalen Vorrichtungsende zugewandten Seite eine von seinem Außenumfang in Richtung zur Vorrichtungsmittelachse verlaufende Abschrägung (19) aufweist.

5. Vorrichtung nach Anspruch 4, **dadurch gekennzeichnet, dass** die Abschrägung (19) einen Winkel von ≤45° bezogen auf die Vorrichtungsmittelachse bildet.

6. Vorrichtung nach einem der Ansprüche 1 bis 5,
**dadurch gekennzeichnet, dass** die stabförmige Nadel- und Fadenführungseinrichtung (1) in ihrem Zwischenraumbereich (4) eine Durchgangsöffnung (20) aufweist, die mit einer in der stabförmigen Nadel- und Fadenführungseinrichtung (1) vorhandenen und zu deren proximalen Ende hin verlaufenden Anschlussöffnung (21) verbunden ist.

7. Vorrichtung nach Anspruch 6, **dadurch gekennzeichnet, dass** das längliche Außenglied (5) ebenfalls eine Durchgangsöffnung (20') aufweist, die in der den genannten Zwischenraumbereich (4) der stabförmigen Nadel- und Fadenführungseinrichtung (1) verschließenden Stellung des länglichen Außengliedes (5) mit der in dem Zwischenraumbereich (4) der stabförmigen Nadel- und Fadenführungseinrichtung (1) befindlichen Durchgangsöffnung (20) verbunden ist.

8. Vorrichtung nach einem der Ansprüche 1 bis 7,
**dadurch gekennzeichnet, dass** die stabförmige Nadel- und Fadenführungseinrichtung (1) einen ovalförmigen Querschnitt aufweist und dass der Nadelabgabebereich (2) und der Nadelaufnahmebereich (3) lediglich nahe der Endbereiche auf der größeren Querschnittsachse des betreffenden ovalförmigen Querschnitts angeordnet sind.

9. Vorrichtung nach einem der Ansprüche 1 bis 7,
**dadurch gekennzeichnet, dass** die stabförmige Nadel- und Fadenführungseinrichtung (1) einen kreisförmigen Querschnitt oder einen an diesen angenäherten Querschnitt aufweist und dass der Nadelabgabebereich mit entsprechenden Nadelführungsöffnungen und der Nadelaufnahmebereich mit entsprechenden Nadelaufnahmeöffnungen um den betreffenden Querschnitt herum verteilt angeordnet sind.

10. Vorrichtung nach einem der Ansprüche 1 bis 9,
**dadurch gekennzeichnet, dass** das längliche Außenglied (5) mit einer innerhalb oder außerhalb der Nadel- und Fadenführungseinrichtung (1) vorgesehenen Schiebeeinrichtung (17, 23) verbunden und mittels dieser vom proximalen Vorrichtungsende aus in Längsrichtung der Nadel- und Fadenführungseinrichtung (1) verschiebbar ist.

## Claims

1. Surgical device for guiding at least two suture threads (10) through the edge region of a tissue opening, particularly one present in the artery of an individual, if applicable formed by an incision, and for retracting the suture thread ends guided through the edge region in question, in each instance, from the stated opening, having a rod-shaped needle and thread guidance device (1), in which suture threads attached to needles, in each instance, are guided in guidance and/or accommodation openings (15), in such a manner that these can be moved from a needle dispensing region (2) disposed at the proximal end of the device to a needle accommodation region (3) at the distal end of the device, by way of an intermediate space region (4),
whereby the intermediate space region (4) in question has a length that at least corresponds to the thickness of the wall of the tissue opening, and an outer shape, with reference to the outer shape of the needle dispensing region (2) and/or the needle accommodation region (3), that is reduced for unhindered movement of the needles (8) in question between the needle regions in question,
whereby the stated needles (8) can be moved to the needle accommodation region (3), for permanent accommodation there, after having been guided through the edge region of the tissue opening in question,
and whereby afterwards, the rod-shaped needle and thread guidance device (1), together with the needles (8) permanently accommodated in the needle accommodation region (3), and the suture thread ends connected with them, can be retracted from the stated tissue opening again, in such a manner that the latter can be closed by means of drawing together all of the suture thread ends, outside of the tissue opening in question,
**characterized in that** the needle dispensing region (2), the intermediate space region (4), and the needle accommodation region (3) of the rod-shaped needle and thread guidance device (1) are rigidly connected with one another,
that the rod-shaped needle and thread guidance device (1) can be closed off by an elongated outer member (5) that surrounds the intermediate space region (4) with shape fit, which member can be displaced relative to the rod-shaped needle and thread guidance device (1), in its longitudinal direction, releasing the stated intermediate space region (4),
and that the needle dispensing region (2) has an end side (6) towards the distal end of the device, and the elongated outer member has an end side (7) in its region towards the proximal end of the device, which end sides (6, 7) face one another.

2. Device according to claim 1, **characterized in that** the end sides (6, 7) in question run parallel to one another, or at a right angle to the longitudinal direction of the rod-shaped needle and thread guidance device (1).

3. Device according to claim 1 or 2, **characterized in that** the needle accommodation openings (15) of the needle accommodation region (3) are configured in funnel shape in their needle inlet region (18), in each instance.

4. Device according to one of claims 1 to 3, **characterized in that** the needle accommodation region (3) has a bevel (19) that runs from its outside circumference in the direction towards the center axis of the device, on its side facing the proximal end of the device.

5. Device according to claim 4, **characterized in that** the bevel (19) forms an angle of ≤ 45° with reference to the center axis of the device.

6. Device according to one of claims 1 to 5, **characterized in that** the rod-shaped needle and thread guidance device (1) has a passage opening (20) in its intermediate spaceregion (4), which opening is connected with a connection opening (21) present in the rod-shaped needle and thread guidance device (1) that runs towards its proximal end.

7. Device according to claim 6, **characterized in that** the elongated outer member (5) also has a passage opening (20'), which is connected with the passage opening (20) situated in the intermediate space region (4) of the rod-shaped needle and thread guidance device (1) in the position of the elongated outer member (5) that closes off the aforementioned intermediate space region (4) of the rod-shaped needle and thread guidance device (1).

8. Device according to one of claims 1 to 7, **characterized in that** the rod-shaped needle and thread guidance device (1) has an oval cross-section, and that the needle dispensing region (2) and the needle accommodation region (3) are disposed close to the end regions only on the larger crosssection axis of the oval-shaped cross-section in question.

9. Device according to one of claims 1 to 7, **characterized in that** the rod-shaped needle and thread guidance device (1) has a circular cross-section or a cross-section that approximates the latter, and that the needle dispensing region with corresponding needle guidance openings and the needle accommodation region with corresponding needle accommodation openings are disposed distributed around the cross-section in question.

10. Device according to one of claims 1 to 9, **characterized in that** the elongated outer member (5) is connected with a pusher device (17, 23) provided within or outside of the needle and thread guidance device (1), and can be displaced in the longitudinal direction of the needle and thread guidance device (1), from the proximal end of the device, by means of this device.

## Revendications

1. Dispositif chirurgical destiné à guider au moins deux fils de suture (10) à travers la zone marginale d'une ouverture pratiquée dans le tissu, en particulier dans une artère d'un individu et réalisée le cas échéant par incision, et à retirer de ladite ouverture les extrémités respectives des fils de suture qui ont été menées à travers la zone marginale respective, comprenant un dispositif de guidage d'aiguille et de fil (1) en forme de tige à l'intérieur duquel des fils (10) de suture fixés respectivement sur des aiguilles (8) sont guidés dans des ouvertures de guidage (15) ou bien de réception de manière à pouvoir être déplacés d'une zone de distribution d'aiguille (2) située à l'extrémité proximale du dispositif vers une zone de réception d'aiguille (3) située à l'extrémité distale du dispositif, en passant par une zone d'espacement (4), ladite zone d'espacement (4) respective présentant une longueur correspondant au moins à l'épaisseur de la paroi de ladite ouverture pratiquée dans le tissu, et ayant une forme extérieure réduite par rapport à la forme extérieure de ladite zone de distribution d'aiguille (2) et/ou de la zone de réception d'aiguille (3), pour un mouvement libre des aiguilles (8) respectives entre les zones d'aiguille respectives, lesdites aiguilles (8) - après les avoir menées à travers la zone marginale de l'ouverture respective dans le tissu - pouvant être déplacées dans la zone de réception d'aiguille (3) pour y être reçues en permanence, et, ensuite, ledit dispositif de guidage d'aiguille et de fil (1) en forme de tige conjointement avec les aiguilles (8) reçues en permanence dans la zone de réception d'aiguille(3) ainsi qu'avec les extrémités y reliées des fils de suture pouvant être retirés de ladite ouverture dans le tissu, de telle manière que l'ouverture respective pratiquée dans le tissu peut être refermée en rassemblant l'ensemble des extrémités de fils de suture à l'extérieur de ladite ouverture, **caractérisé en ce que** la zone de distribution d'aiguille (2), la zone d'espacement (4) et la zone de réception d'aiguille (3) du dispositif de guidage d'aiguille et de fil (1) en forme de tige sont reliées rigidement entre elles, **que** ledit dispositif de guidage d'aiguille et de fil (1) en forme de tige peut être obturé par un élément extérieur allongé (5) qui embrasse à engagement positif sa zone d'espacement (4) et qui peut être déplacé par rapport au dispositif de guidage d'aiguille et de fil (1) en forme de tige, dans la direction longitudinale de celui-ci, en dégageant ladite zone d'espacement (4), **et que** ladite zone de distribution d'aiguille (2), à savoir en direction de l'extrémité distale du dispositif, et ledit élément extérieur allongé (5), à savoir dans sa zone montrant en direction de l'extrémité proximale du dispositif, présentent des faces terminales (6, 7) montrant respectivement l'une vers l'autre.

2. Dispositif selon la revendication 1, **caractérisé en ce que** les faces terminales respectives (6, 7) s'étendent parallèlement l'une à l'autre ou à angle droit par rapport à la direction longitudinale du dispositif de guidage d'aiguille et de fil (1) en forme de tige.

3. Dispositif selon la revendication 1 ou 2, **caractérisé en ce que** les ouvertures de réception d'aiguille (15) de ladite zone de réception d'aiguille (3) sont réalisées dans leur zone respective d'entrée d'aiguille (18) de manière à présenter une forme d'entonnoir.

4. Dispositif selon l'une quelconque des revendications 1 à 3, **caractérisé en ce que,** sur sa face tournée vers l'extrémité proximale du dispositif, ladite zone de réception d'aiguille (3) présente un biais (19) s'étendant depuis sa circonférence extérieure en direction de l'axe médian du dispositif.

5. Dispositif selon la revendication 4, **caractérisé en ce que** ledit biais (19) forme un angle ≤ 45° par rapport à l'axe médian du dispositif.

6. Dispositif selon l'une quelconque des revendications 1 à 5, **caractérisé en ce que**, dans sa zone d'espacement (4), le dispositif de guidage d'aiguille et de fil (1) en forme de tige présente une ouverture de passage (20) qui communique avec une ouverture de raccordement (21) présente dans le dispositif de guidage d'aiguille et de fil (1) en forme de tige et s'étendant vers l'extrémité proximale de celui-ci.

7. Dispositif selon la revendication 6, **caractérisé en ce que** ledit élément extérieur allongé (5) présente, lui aussi, une ouverture de passage (20') qui, lorsque l'élément extérieur allongé (5) se trouve dans la position où elle ferme ladite zone d'espacement (4) du dispositif de guidage d'aiguille et de fil (1) en forme de tige, communique avec ladite ouverture de passage (20) réalisée dans la zone d'espacement (4) du dispositif de guidage d'aiguille et de fil (1) en forme de tige.

8. Dispositif selon l'une quelconque des revendications 1 à 7, **caractérisé en ce que** ledit dispositif de guidage d'aiguille et de fil (1) en forme de tige présente une section transversale ovale et que la zone de distribution d'aiguille (2) et la zone de réception d'aiguille (3) sont disposées uniquement à proximité des zones terminales sur l'axe de section plus grand de la section ovale respective.

9. Dispositif selon l'une quelconque des revendications 1 à 7, **caractérisé en ce que** ledit dispositif de guidage d'aiguille et de fil (1) en forme de tige présente une section transversale circulaire ou une section transversale proche de celle-ci, et que la zone de distribution d'aiguille avec des ouvertures correspondantes de guidage d'aiguille ainsi que la zone de réception d'aiguille avec des ouvertures correspondantes de réception d'aiguille sont disposées de manière à être réparties autour de la section respective.

10. Dispositif selon l'une quelconque des revendications 1 à 9, **caractérisé en ce que** ledit élément extérieur allongé (5) est relié à un dispositif de poussée (17, 23) prévu à l'intérieur ou à l'extérieur du dispositif de guidage d'aiguille et de fil (1) et peut être déplacé par le biais de ce dispositif de poussée à partir de l'extrémité proximale du dispositif dans la direction longitudinale du dispositif de guidage d'aiguille et de fil (1).
